# EUROPEAN PATENT SPECIFICATION

(11) **EP 4 478 916 B1**
(45) Date of publication and mention of the grant of the patent: **06.05.2026**
(21) Application number: 23913859.7
(22) Date of filing: 19.12.2023
(51) Int. Cl.: A47G 9/02, A61F 7/00, A61F 7/02, D04H 1/558, D04H 1/413, D04H 1/74, B32B 5/02, B32B 27/12, B32B 27/32

(54) **PRODUCTION METHOD FOR A BABY COVER**
HERSTELLUNGSVERFAHREN FÜR EINE BABYHÜLLE
PROCÉDÉ DE FABRICATION D'UNE COUVERTURE POUR BÉBÉ

(30) Priority: 11.05.2023 TR 202305264
(43) Date of publication of application: 25.12.2024
(73) Proprietor: Pelsan Tekstil Urunleri Sanayi ve Ticaret Anonim Sirketi, 34522 Istanbul (TR); Telasis Tekstil Urunleri Sanayi ve Ticaret Anonim Sirketi, 34522 Istanbul (TR)
(72) Inventor: ÜSTÜN AYDIN, Hüner, 34522 Istanbul (TR)
(74) Representative: Kayahan, Senem
(86) International application number: PCT/TR2023/051590
(87) International publication number: WO 2024/232844

(56) References cited:
- CN-A- 105 670 569
- CN-U- 208 657 990
- KR-A- 20150 007 916
- US-A1- 2015 066 119
- US-A1- 2016 374 411
- US-B2- 9 894 944

## Description

### Technical Field

The invention is related to the production method for a baby cover containing a breathable film laminated with a nonwoven, which visually indicates the increase in body temperature in babies to parents through the thermochromic indicator on its surface and provides a refreshing effect to babies with the help of microcapsules.

### State of the Art

In particular, newborn babies and children in their first year can get fever for a variety of reasons. In the present art, the cover materials used for babies during fever are usually produced using the spunlace (water jet) method. Nonwoven surfaces produced by the spunlace method are textile surfaces obtained by entangling the fibers forming the nonwoven surface with pressurized water which is used in the production of wet wipes and disposable medical textile products. However, the spunlace method causes lamination problems. In addition, the present art lacks microcapsule support. The products are only laminated with pure nonwovens.

A search of the present art revealed the application no. TR2013/08704. The application relates to an embodiment of a baby blanket that provides thermal insulation. The embodiment of the blanket/cover comprises a protective outer layer, a silicone layer for thermal insulation and a closure arrangement. However, the application does not include a non-woven thermal bonded baby cover containing a laminated breathable film and microcapsules.

A baby cover suitable for cooling a baby in order to provide relief from a fever is disclosed in CN 208 657 990 U.

As a result, due to the above-mentioned drawbacks and the shortcomings of the existing solutions, a development in the relevant technical field is required.

### The Object of the Invention

The invention is inspired by existing situations and aims to solve the above-mentioned problems.

The main object of the invention is to refresh the baby with the baby cover obtained by a method comprising a step of laminating a thermobonded nonwoven surface layer comprising microcapsules with a breathable film, and to visually indicate the body temperature of the baby to the parents through the thermochromic indicator, as defined in claim 1. Further embodiments are defined in the dependent claims.

The object of the invention is to provide a thermobonded and carded baby cover with a silk, soft and coolness effect, which has an increased breathing capacity with a nonwoven surface, as defined in claim 9.

The object of the invention is to ensure contact and data acquisition from more spots by increasing the printing area and effectiveness of the thermochromic indicator.

In the invention, instead of nonwoven surface layers produced with water jet, a thermbonded and carded nonwoven surface layer is used, which is more rigid, smooth, does not reduce the breathing capacity of the film and provides a soft and silk effect. In addition, functionality is increased by impregnating the nonwoven surface with microcapsules for breath easy and refreshing.

The invention provides a baby cover with increased breathing capacity as well as an increased printing area of the thermochromic indicator.

The invention is made entirely from bio-based materials. The baby cover according to the invention comprises low-density polyethylene (LDPE) and is derived from renewable sugar cane. LDPE is a 100% recyclable and compostable eco-friendly resource that absorbs carbon dioxide from the atmosphere. According to the invention the baby cover can also be produced from biopolymers containing completely biodegradable PLA and PBAT.

The invention used MDO (Machine Direction Orientation) technology for breathability.

With this technology, during the polyethylene film production process, the polyethylene film is stretched longitudinally by passing it through a machine, thus making the film more durable, stronger and with a smooth surface structure. MDO technology also enables the production of thinner and lighter products (films) during film production. In this way, environmentally friendly and economical production can be achieved in the film industry. This provides breathing properties to the cover.

The nonwoven surface layer included in the invention is produced from soybean protein fibers (SPF), biofibers and green bicomponent fibers. Besides being biodegradable, these fibers are the only renewable botanical protein fibers. SPF provides the subject of the invention with nourishing properties for healthy skin owing to the amino acid and protection from ultraviolet rays. Green bicomponent fibers provide biobased solutions and are produced from sugarcane.

The structural and characteristic features and all advantages of the invention will be more apparent from the following figures and the detailed description with reference to these figures and therefore the interpretation should be based on these figures and the detailed description.

### Figures to Understand the Invention

**Fig. 1** is a representative view of the assembly in which the production of the thermobonded nonwoven fabrics is performed.
**Fig. 2** is a representative view of the assembly in which the foulard method is performed.

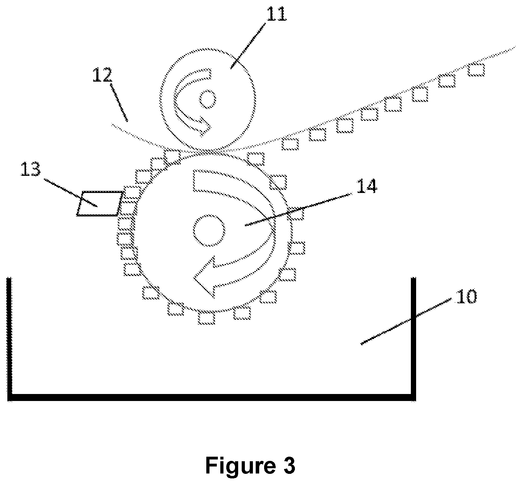

**Fig. 3** is a representative view of the assembly in which the rotogravure printing method is performed.

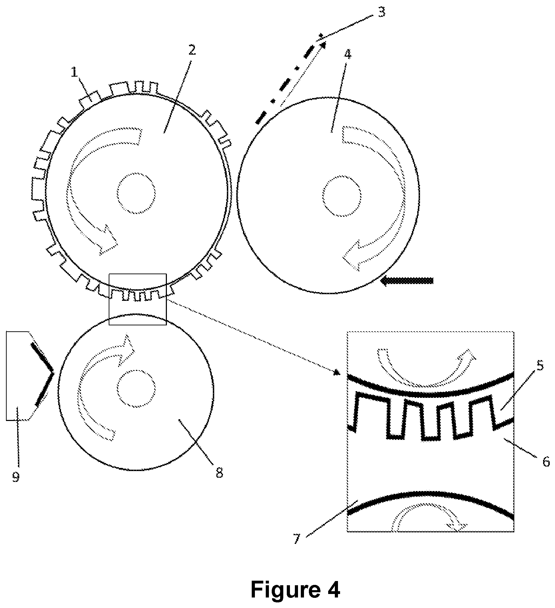

**Fig. 4** is a representative view of the assembly in which the flexo printing method is performed.

### Description of Part References

1. Fiber
2. Opening unit
3. Feeding unit
4. Carding unit
5. Calender unit
6. Bonding unit
7. Immersion cylinder
8. Foulard tank
9. Mangle cylinder
10. Indicator chamber
11. Cliché plate cylinder
12. Underprint material
13. Scraper
14. Gravure cylinder

### Detailed Description of the Invention

In this detailed description, the preferred embodiments of the production method for a baby cover according to the invention are described only for a better understanding of the subject matter.

The baby cover according to the invention can be made by combining a thermochromic indicator-printed breathable polyethylene film and a microcapsule-impregnated, carded, thermobonded nonwoven surface layer with rubber-based adhesive. The method according to the invention comprises the process steps of:
- production of a carded, thermobonded nonwoven surface layer and
   impregnation of the surface layer with microcapsules using a foulard method,
- production of a thermochromic indicator-printed polyethylene film derived from sugar cane or a PLA or PBAT biofilm, produced using blown film technology and machine direction orientation.
- combining the surface layer and the film by a lamination process.

The microcapsule impregnation process is carried out by a foulard method. The microcapsules impregnated with the foulard method are penetrated into the nonwoven surface and explode when in contact with the body, producing a cooling and refreshing effect. Production of a carded, thermobonded nonwoven surface layer provides a silky and cost-effective product that allows for different fiber (1) blends. In the method, the printing method is applied for thermal indicator. The thermochromic indicator becomes invisible when the body temperature rises above 37 degrees and becomes visible again when the body temperature returns to normal.

The polyethylene film used as the bottom layer of the baby cover product is called 'Green Polyethylene Film'. The reason it is called 'green' is that the raw material for the film, polyethylene, is derived from sugar cane which is a renewable resource instead of fossil resources. In terms of film properties, green polyethylene offers the properties of films produced from fossil resources. In addition, green polyethylene is biodegradable and compostable. This makes it environmentally friendly. For example, one ton of green polyethylene can absorb up to 3.9 tons of CO2 from the atmosphere. Thus, it helps to reduce the harmful greenhouse gas impact. In addition, products made from green polyethylene are 100% recyclable within ten years of their lifetime. Also PLA and PBAT blended biofilms can be used as a bottom layer of the baby cover product. In terms of biofilm properties PLA and PBAT provide 100% compostable and biodegradable solutions.

The green polyethylene film and 100% biofilm are produced by the blowing technology.

Firstly, polymers, compounds, additives and MDO (machine direction orientation) technology were used to provide breathability when producing the film on the blowing line.

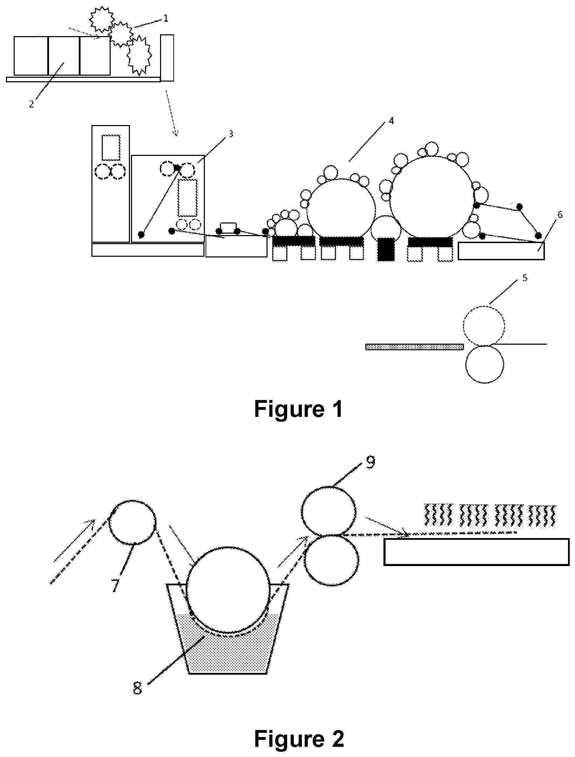

Fig. 1 shows a representative view of the assembly in which the production of the thermobonded nonwoven surface layer is performed. In the production of thermobonded nonwovens; synthetic staple, regenerated, bicomponent or natural fibers with different cross-sectional structures are blended and nonwovens obtained by carding, dry laying and thermal bonding processes are used. Thermobonded nonwoven production consists of the following stages:
- pre-opening of fibers (1) with different cross-sections and structures in the fiber opening unit (2),
- mixing the opened fibers (1) with aeration in the feeding unit (3),
- carding of the fibers (1) by means of cylinders and drums,
- combining of the carded fibers (1) in the bonding unit (6) and calendering in the calender unit (5) at high temperature and pressure,
- fixing the calendered fibers (1) through thermal welding points to obtain the thermobonded nonwoven surface layer.

### Fiber Opening Process

At this stage, synthetic staple, regenerated or natural (1) with different cross-sectional structures are pre-opened in the fiber opening unit (2), preferably mechanically, in single or octuple blends with ratios in the range of 1-100%. Then the collector is delivered to the mixer cylinders and the fiber (1) is sent to the storage.

### Fiber Feeding Process

At this stage, the opened fibers (1) are delivered to the storage and then to the feeding unit (3). Here, fibers (1) of different types and structures are mixed in the desired blend with aeration. Via the conveyor belt, the fibers (1) are sent to the feeding cylinders and then laid on the scaled conveyor belt for the carding process.

### Carding Process

At this stage, the fiber (1) mixture, preferably in the range of 8 g/m² - 100 g/m², delivered by a scaled conveyor belt, is opened by ±450 and laid randomly in parallel and cross directions in a system consisting of cylinders and drums organized in different diameters, at different speeds, in different directions, with different technical equipment; and directed to the belt system with a different number of transfer drums. The equipment properties of the cylinders that will perform the carding process, the structure-slope and placement of these equipment, the revolutions of the cylinders take values depending on variables such as raw material fiber (1) mixture, fiber (1) denier-length values, and the morphological structure of the fiber. Fiber (1) features should preferably be in the range of 0.5-15 denier and 30-80 mm length. Fibers (1) can be mono or bicomponent synthetic fibers such as polyester, polyamide, polypropylene, etc., bicomponent synthetic fibers with different cross-sectional structures, such as polyethylene-polypropylene, polyester-copolyester, etc., synthetic fibers with different cross-sectional structures such as round-hollow-trilobal etc., natural and regenerated fibers such as viscose, cotton, etc. The choice of these fibers, their mixture and mixing ratios are crucial for the parameters regarding the equipment properties of the cylinders.

### Bonding Process

At this stage, the carded fibers (1) obtained as a result of the carding stage are organized and combined into a single web on the transfer belt. The carded web is delivered to the internally oil heated calender unit (5). The fibers (1) are passed between a hot flat calender and a hot patterned calender at the temperature, pressure and speed values suitable for the mixture thereof. The carded web is calendered with high temperature and pressure, fixed through thermal welding points and the carded thermobonded nonwoven is formed. Although the parameters vary according to the raw materials, it is preferred that the temperature values are in the range of 35-500 C°, the temperature difference between the calenders is at values of ±20 C° and the patterned calender thermal bonding area is in the range of 5-40% mm2.

As a result of the process steps of fiber opening, fiber feeding, carding and bonding, the details of obtaining the thermobonded nonwoven surface layer is provided above. The mentioned process steps can be completed manually or with different equipment that will perform the same function.

The process parameters are crucial for effective results. The bonding process in particular is critical to the invention. At this stage, the carded web passes between the calenders and the thermal bonding process takes place.

The range of fibers that can be used in the fiber (1) opening process, which is the first stage of the process according to the invention, is considerably wide. However, in the state of the art, the fibers (1) that can be worked with are limited to spunlace, spunbond and meltblown.

### Microcapsule Application (Foulard System)

The state of the art does not include microcapsule application (foulard system) and the refreshing microcapsule support available in the new product does not exist. The incoming nonwoven surface is transferred to the foulard tank (8) with the immersion cylinder (7) and meets the liquid containing the microcapsules. Impregnation is carried out with 0.5-300 grams per m2. The excess amount is removed with the mangle cylinder (9) and the drying process is started.

### Production Method for Polyethylene Film

Blown film technology is a very common form of film production in which polyethylene and other raw materials are first melted in an extruder and mixed and then the balloon inflation method is applied in a blowing head for the production. As the film begins to move away from the head, it begins to cool. The film coming down from the tower enters the orientation unit (MDO) (machine direction orientation). It is slightly heated at the beginning of the unit, then stretched. During this stretching, the micropores in the film open and the film becomes breathable. The film that comes down and wound on the winder is ready to be used in the required area.

### Indicator Printing Method

Rotogravure printing method: The printing technique used in the invention is performed by filling the prepared indicator into the indicator chamber (10) and transferring it to the film surface by pouring indicator onto the cliché plate cylinder (11) (cylinder machined on the metal cylinder by the abrasion method). It is then specifically prepared in the shapes to be printed.

Flexo printing method: The cliché plates used for this printing technique are flexible. In this printing process, the printing area must be at a certain height from the plate area, depending on the process. The flexible cliché plate (mould) can be easily bent. The cliché plate is mounted on the mould cylinder using double-sided tape. The anilox rotates in the indicator chamber (10) and the indicator is loaded into the cell pits of the anilox. A scraper scrapes the indicator loaded into the high parts of the anilox. The indicator remaining in the cell cavities is transferred to the high parts of the printing cliché plate. Then, when the material to be printed passes between the gravure cylinder (14) and the cliché plate cylinder (11), the printing process is applied with the indicator remaining on the surface.

### Hot-melt Lamination

The method of adhering two separate materials such as Film, Spunbond, Spunlace, CTB Non-woven, etc. with hot adhesive is called "Hot-melt Lamination". The fabrics, films and adhesives used in lamination can have different properties depending on the intended use of the product. The hot-melt lamination process is carried out in order to provide visual and aesthetic properties, to increase technical performance, to differentiate functional properties and to obtain additional properties.

The method of the invention is based on the CTB nonwoven and breathable film lamination with hot melt rubber-based adhesive.

## Claims

1. A production method for a baby cover which has a fever indicator and provides a cooling and refreshing effect to the baby, **characterized in that** it comprises the steps of:
a. production of a carded, thermobonded nonwoven surface layer obtained from synthetic staple, regenerated, bicomponent or natural fibers and impregnation of the surface with microcapsules capable of producing a cooling and refreshing effect when they burst following contact with the baby's body, using foulard method,
b. production of a thermochromic indicator-printed breathable polyethylene film derived from sugar cane, or a breathable PLA or PBAT biofilm, produced using blown film technology and machine direction orientation,
c. combining the surface of step a and the film of step b by a lamination process.

2. The production method for a baby cover according to claim 1, **characterized in that** the thermobond nonwoven production comprises the steps of:
• pre-opening of fibers (1) with different cross-sections and structures in the fiber opening unit (2),
• mixing the opened fibers (1) with aeration in the feeding unit (3),
• carding of the fibers (1) by means of cylinders and drums,
• combining of the carded fibers (1) in the bonding unit (6) and calendering in the calender unit (5) at high temperature and pressure,
• fixing the calendered fibers (1) through thermal welding points to obtain the thermobond non-woven surface.

3. The production method for a baby cover according to claim 1, **characterized in that** the foulard method comprises the steps of:
• transferring the non-woven surface to the foulard tank (8) with the immersion cylinder (7) and immersing it in the liquid containing the microcapsules,
• removing the excess liquid on the surface with a mangle cylinder (9) and drying the surface.

4. The production method for a baby cover according to claim 1, **characterized in that** production of film of step b comprises the steps of:
• melting the polyethylene and raw materials in the extruder, mixing them, passing them through the film blowing head, cooling, stretching and solidifying,
• providing the film with breathing properties by opening the micropores in the film as the solidified film enters the elastomerization and orientation unit in the machine production direction.

5. The production method for a baby cover according to claim 4, **characterized in** further comprises step of thermochromic indicator-printing selected from rotogravure and flexo printing method.

6. The production method for a baby cover according to claim 5, **characterized in that** rotagravur printing method comprises, t filling the prepared indicator into the indicator chamber (10) and transferring it to the film surface by pouring indicator onto the cliché plate cylinders (11).

7. The production method for a baby cover according to claim 5, **characterized in that** flexo printing method comprises steps of; mounting the cliché plate on mould cylinder, rotating anilox in the indicator chamber (10),loading the indicator into cell pits of the anilox and and applying the indicator printing process to the surface between the gravure cylinder (14) and the cliché plate cylinder (11).

8. The production method for a baby cover according to claim 1, **characterized in that** the lamination process is applied with hot-melt rubber-based adhesive.

9. A baby cover obtainable by the method according to one of claims 1 to 8.

## Patentansprüche

1. Eine Produktionsmethode für eine Babyabdeckung, die einen Fieberindikator hat und dem Baby eine kühlende sowie erfrischende Wirkung verleiht, gekennzeichnet darin, dass sie aus folgenden Schritten besteht:
a. Herstellung einer kardierten, thermogebundenen, nicht gewebten Oberflächenschicht, die aus synthetischem Stapelmaterial stammt, regenerierten, bikomponentigen oder natürlichen Fasern gewonnen und die Oberfläche mit Mikrokapseln impregniert wird, die beim Platzen nach Kontakt mit dem Körper des Babys eine kühlende und erfrischende Wirkung erzeugen können, unter Verwendung der Foulard-Methode.
b. b. Produktion von a Thermochromatisch Blinker-gedruckt atmungsaktiver Polyethylenfilm aus Zuckerrohr oder ein atmungsaktiver PLA- oder PBAT-Biofilm, der mit Blasfilmtechnologie und Maschinenrichtung hergestellt wird,
c. indem die Oberfläche von Schritt A und der Film von Schritt B durch ein Laminierungsverfahren kombiniert werden.

2. Das Herstellungsverfahren für eine Babyabdeckung gemäß Anspruch 1 ist **dadurch gekennzeichnet, dass** die thermobindende Nonwoven-Produktion aus folgenden Schritten besteht:
• Voröffnung von Fasern (1) mit unterschiedlichen Querschnitten und Strukturen in der Faseröffnungseinheit (2),
• Mischung der geöffneten Fasern (1) mit Belüftung in der Zuführeinheit (3),
• Kardierung der Fasern (1) mittels Zylindern und Trommeln,
• durch Kombination der gecardeten Fasern (1) in der Bindungseinheit (6) und des Kalenders in der Kalendereinheit (5) bei hoher Temperatur und hohem Druck,
• Fixieren der kalendierten Fasern (1) durch thermische Schweißpunkte, um die thermobindende nicht-gewebte Oberfläche zu erhalten.

3. Das Herstellungsverfahren für eine Babyabdeckung gemäß Anspruch 1, **gekennzeichnet dadurch, dass** die Foulard-Methode die folgenden Schritte umfasst:
• wobei die nicht gewebte Oberfläche mit dem Immersionszylinder (7) in den Foulard-Tank (8) übertragen und in die Flüssigkeit mit den Mikrokapseln eingetaucht wird,
• Entfernen der überschüssigen Flüssigkeit auf der Oberfläche mit einem zerschmetterten Zylinder (9) und das Trocknen der Oberfläche.

4. Die Herstellungsmethode für einen Babybezug gemäß Anspruch 1, charakterisiert durch die Herstellung von Film von Schritt b, umfasst die folgenden Schritte:
• das Polyethylen und die Rohmaterialien im Extruder schmelzen, mischen, durch den Blaskopf der Filmfolie führen, abkühlen, dehnen und verhärten,
• indem der Film durch das Öffnen der Mikroporen im Film beim Eintreten der Elastomerisierungs- und Orientierungseinheit in die Elastomerisierungs- und Orientierungseinheit in der Produktionsrichtung der Maschine verliehen wird.

5. Das Produktionsverfahren für eine Babybekleidung gemäß Behauptung 4, charakterisiert im weiteren Fall, umfasst den Schritt der thermochromen Indikatordruck wurde aus der Rotogravur- und Flexodruckmethode ausgewählt.

6. Die Herstellungsmethode für einen Babycover gemäß Anspruch 5, charakterisiert durch das Rotagravur-Druckverfahren, besteht darin, den vorbereiteten Indikator in die Indikatorkammer (10) zu füllen und ihn durch Gießen auf die Klischee-Plattenzylinder auf die Filmoberfläche zu übertragen (11).

7. Das Herstellungsverfahren für eine Babyabdeckung gemäß Anspruch 5, charakterisiert durch dieses Flexodruckverfahren, besteht aus folgenden Schritten; Montage der Klischeeplatte auf dem Formzylinder, rotierende Aniloxe in der Indikatorkammer (10), Laden des Indikators in die Zellgruben der Aniloxe und Anwendung des Indikatordruckprozesses auf der Oberfläche zwischen dem Gravurezylinder (14) und dem Klischee-Plattenzylinder (11).

8. Die Herstellungsmethode für eine Babyabdeckung nach Anspruch 1 ist **dadurch gekennzeichnet, dass** der Laminierungsprozess mit heißschmelzendem Gummikleber aufgetragen wird.

9. Eine Kinderdecke, die durch die Methode gemäß einem der Ansprüche 1 bis 8 erhältlich ist.

## Revendications

1. Une méthode de production pour une housse pour bébé qui possède un indicateur de fièvre et procure un effet rafraîchissant et rafraîchissant au bébé, **caractérisée par** les étapes suivantes suivantes :
a. la production d'une couche de surface cardée, thermoliée, non tissée, obtenue à partir d'agrafes synthétiques, régénérées, bicomposantes ou naturelles, et l'imprégnation de la surface avec des microcapsules capables de produire un effet rafraîchissant et rafraîchissant lorsqu'elles éclatent après le contact avec le corps du bébé, en utilisant la méthode Foulard,
b. b. Production de a Thermochromique Imprimé par indicateurs Film de polyéthylène respirant dérivé de la canne à sucre, ou un biofilm PLA ou PBAT respirant, produit grâce à la technologie du film soufflé et à l'orientation de la direction,
c. Combinant la surface de l'étape A et le film de l'étape B par un procédé de lamination.

2. La méthode de production d'une housse pour bébé selon la revendication 1, **caractérisée par** la production non tissée thermoliant comprend les étapes suivantes :
• pré-ouverture des fibres (1) avec différentes sections transversales et structures dans l'unité d'ouverture de la fibre (2),
• mélanger les fibres ouvertes (1) avec l'aération dans l'unité d'alimentation (3),
• cardage des fibres (1) au moyen de cylindres et de tambours,
• la combinaison des fibres cardées (1) dans l'unité de liaison (6) et le calandre dans l'unité de calandre (5) à haute température et pression,
• fixation des fibres calandres (1) par des points de soudure thermique pour obtenir la surface thermoliaison non tissée.

3. La méthode de production d'une housse pour bébé selon l'affirmation 1, **caractérisée par** la méthode foulard comprend les étapes suivantes :
• transférer la surface non tissée dans le réservoir foulard (8) avec le cylindre d'immersion (7) et l'immerger dans le liquide contenant les microcapsules,
• Enlever l'excès de liquide à la surface avec un barillet de mangle (9) et sécher la surface.

4. La méthode de production d'une housse pour bébé selon la revendication 1, caractérisée en ce sens que la production de film de l'étape b, comprend les étapes suivantes :
• fondre le polyéthylène et les matières premières dans l'extruseur, les mélanger, les faire passer à travers la tête de soufflage du film, refroidir, étirer et solidifier,
• il confère au film des propriétés respiratoires en ouvrant les micropores du film lorsque le film solidifié entre dans l'unité d'élastomérisation et d'orientation dans la direction de production de la machine.

5. La méthode de production d'une housse pour bébé selon la revendication 4, **caractérisée par** la suite, comprend une étape thermochromique Impression par indicateurs sélectionnée à partir de la rotogravure et de la méthode d'impression flexo.

6. La méthode de production d'une housse pour bébé selon la revendication 5, **caractérisée par** la méthode d'impression rotagravur, consiste à remplir l'indicateur préparé dans la chambre de l'indicateur (10) et à le transférer sur la surface du film en versant l'indicateur sur les cylindres clichés (11).

7. La méthode de production d'une housse pour bébé selon la revendication 5, **caractérisée par** la méthode d'impression flexo, comprend des étapes suivantes : montage de la plaque cliché sur le cylindre du moule, rotation de l'anilox dans la chambre indicatrice (10), chargement de l'indicateur dans les fosses de cellules de l'anilox et application du procédé d'impression de l'indicateur à la surface entre le cylindre de gravure (14) et le cylindre à plaque cliché (11).

8. La méthode de production d'une housse pour bébé selon la revendication 1, **caractérisée par le fait que** le procédé de lamination est appliqué avec un adhésif à base de caoutchouc thermofusible.

9. Une couverture pour bébé disponible selon l'une des revendications 1 à 8.
